(19) Europäisches Patentamt
European Patent Office
Office européen des brevets

(11) Numéro de publication: **0 008 259**
**A1**

(12) **DEMANDE DE BREVET EUROPEEN**

(21) Numéro de dépôt: **79400509.0**

(22) Date de dépôt: **19.07.79**

(51) Int. Cl.³: **C 07 D 401/04, A 61 K 31/445**

(30) Priorité: **20.07.78 CH 7844/78**

(43) Date de publication de la demande: **20.02.80**
**Bulletin 80/4**

(84) Etats contractants désignés: **BE CH DE FR GB IT NL SE**

(71) Demandeur: **SCIENCE UNION ET Cie SOCIETE FRANCAISE DE RECHERCHE MEDICALE, 14 rue du Val d'Or, F-92150 Suresnes (FR)**

(72) Inventeur: **Duhault, Jacques, 14 bis rue Paul Demange, F-78290 Croissy/Seine (FR)**
Inventeur: **Dhainaut, Alain, 16 rue Marice Ravel, F-92230 Gennevilliers (FR)**
Inventeur: **Regnier, Gilbert, Avenue du Plessis, F-92290 Chatenay-Malabry (FR)**
Inventeur: **Boulanger, Michelle, 71, avenue Auguste Renoir, F-78160 Marly-Le-Roi (FR)**

(74) Mandataire: **Reverbori, Marcelle, SCIENCE UNION ET Cie SOCIETE FRANCAISE DE RECHERCHE MEDICALE Service Brevets 22 rue Garnier, F-92200 Neuilly/Seine (FR)**

(54) **Nouveaux dérivés de la pipéridylbenzimidazolinone, leurs procédés de préparation et les compositions pharmaceutiques les renfermant.**

(57) Nouveaux dérivés de la pipéridylbenzimidazolinone, utilisables comme médicament et répondant à la formule générale

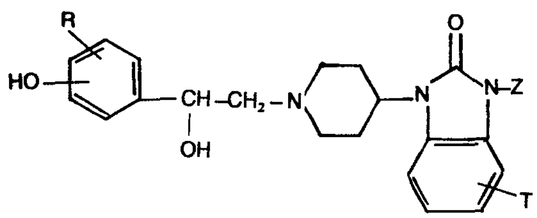

dans laquelle

R est hydrogène, hydroxy, hydroxyméthyle, méthoxycarbonyle, formamido, acétamido, mésylamino, oxalamino, éthoxalylamino, carbamoyle

(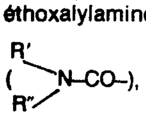), uréido (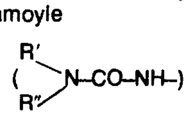) ou sulfamoylamino

(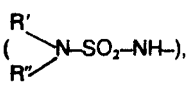), R' et R'' identiques ou différents, étant hydrogène ou alkyle ayant de 1 à 5 atomes de carbone,

T est hydrogène, halogène, ou alkyle ou alkoxy ayant chacun de 1 à 5 atomes de carbone, et

Z est hydrogène, ou alkyle ou alcényle, ayant chacun de 1 à 5 atomes de carbone en chaine droite ou ramifiée.

Ces nouveaux composés et leurs sels physiologiquement tolérables peuvent être utilisés en thérapeutique notamment dans le traitement des maladies autoimmunes, allergiques et inflammatoires et dans le traitement de l'insuffisance respiratoire de l'asthme.

EP 0 008 259 A1

Nouveaux dérivés de la pipéridylbenzimidazolinone, leurs procédés de préparation et les compositions pharmaceutiques les renfermant.

La présente invention a pour objet des dérivés de la pipéridylbenzimidazolinone, leurs procédés de préparation et les compositions pharmaceutiques les renfermant.

Elle concerne particulièrement les dérivés de la pipéridylbenzimidazolinone de formule générale I :

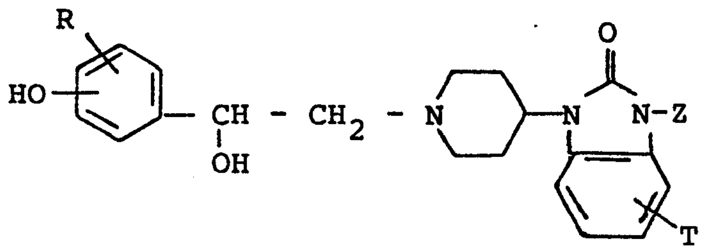

I

dans laquelle :

R représente un atome d'hydrogène, ou un radical hydroxy (OH-), hydroxyméthyle ($HO-CH_2-$), méthoxycarbonyle ($CH_3-O-C(=O)-$), formamido (H-CO-NH-), acétamido ($CH_3-CO-NH-$), mésylamino ($CH_3-SO_2-NH-$), oxalamino (HOOC-CO-NH-), éthoxalylamino ($C_2H_5OOC-CO-NH-$), carbamoyle ($R'R''N-CO-$), uréido ($R'R''N-CO-NH-$) ou sulfamoylamino ($R'R''N-SO_2-NH-$),

R' et R" étant dans ces formules identiques ou différents et représentant chacun un atome d'hydrogène ou un radical alkyle ayant de 1 à 5 atomes de carbone,

T représente un atome d'hydrogène ou d'halogène, ou un radical alkyle ou alkoxy ayant chacun de 1 à 5 atomes de carbone, et

Z représente un atome d'hydrogène, ou un radical alkyle ou alcényle chacun renfermant de 1 à 5 atomes de carbone en chaîne droite ou ramifiée.

La présente invention a également pour objet le procédé de préparation des dérivés de formule générale I caractérisé en ce que :
l'on fait réagir une haloacétophénone de formule générale II

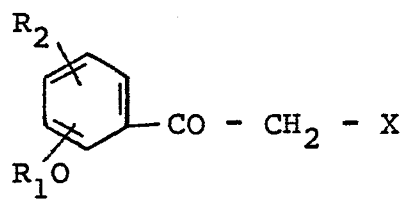

II

dans laquelle :

X représente un atome de chlore ou de brome,

$R_1$ représente un atome d'hydrogène ou un groupe protecteur facilement hydrolysable ou hydrogénolysable tel que par exemple un radical acétyle ou benzyle et

$R_2$ représente un atome d'hydrogène, ou un radical hydroxy, acétoxy, benzyloxy, hydroxyméthyle, acétoxyméthyle, benzyloxyméthyle, méthoxycarbonyle ($CH_3\text{-}O\text{-}\underset{\underset{O}{\|}}{C}\text{-}$), formamido (H-CO-NH-), acétamido ($CH_3$-CO-NH-), mésylamino ($CH_3$-$SO_2$-NH-), oxalamino (HOOC-CO-NH-), éthoxalylamino ($C_2H_5$OOC-CO-NH-), carbamoyle ($\overset{R'}{\underset{R''}{>}}$N-CO-), uréido ($\overset{R'}{\underset{R''}{>}}$N-CO-NH-) ou sulfamoylamino ($\overset{R'}{\underset{R''}{>}}$N-$SO_2$-NH-), R' et R" ayant les définitions énoncées précédemment,
sur une pipéridylbenzimidazolinone de formule III :

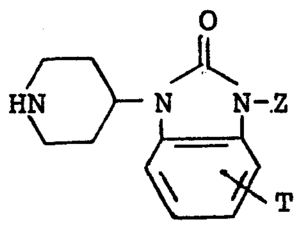

III

dans laquelle

T et Z ont les significations énoncées précédemment,

puis réduit le composé ainsi obtenu de formule générale IV :

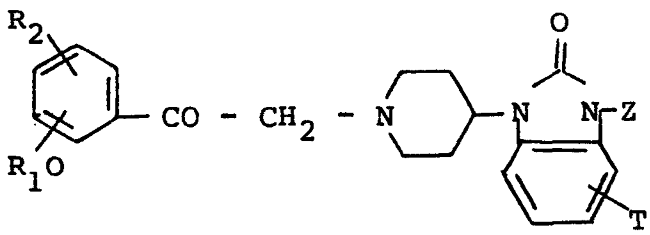

IV

dans laquelle

$R_1$, $R_2$, T et Z ont les définitions énoncées précédemment, et

lorsque le composé réduit renferme des groupes protecteurs, élimine ces derniers selon des méthodes connues.

Il est avantageux d'effectuer la réaction des composés II et III dans un solvant polaire tel qu'un alcool ou une cétone aliphatique ayant 4 ou 5 atomes de carbone ou le diméthyl formamide, à une température comprise entre 110 et 140°C, en présence d'un accepteur de l'hydracide formé au cours de la réaction. Comme accepteurs, on peut utiliser par exemple les carbonates alcalins tel que les carbonates de sodium ou de potassium ou un excès de la pipéridylbenzimidazolinone de formule III.

La réduction du composé IV est accomplie de manière particulièrement adéquate au moyen d'un hydrure alcalin tel que le borohydrure de sodium ($BH_4Na$), ou de potassium ($BH_4K$) ou du cyanoborohydrure de sodium ($BH_3CN$ Na),

dans un solvant polaire tel qu'un alcool aliphatique miscible à l'eau ou le tétrahydrofuranne. Cette réduction peut également être avantageusement réalisée par l'hydrogène sous une pression comprise entre 5 et 70 atmosphères en présence de métaux du 8ème groupe comme catalyseurs tels que le palladium sur charbon, le platine, ou le nickel, dans un solvant polaire tel qu'un alcool aliphatique miscible à l'eau,à une température comprise entre 25 et 80°C.

Le ou les groupes protecteurs éventuellement présents dans le composé de réduction obtenu, peuvent être ensuite éliminés, selon des méthodes connues, soit par hydrolyse acide ménagée (notamment pour le groupe acétyle), ou par hydrogénolyse en présence d'un catalyseur comme par exemple le charbon palladié ou le platine, sous atmosphère d'hydrogène (pour le groupe benzyle).

Les haloacétophénones de formule générale II sont soit connues et décrites dans la littérature, soit préparées selon la méthode décrite par LARSEN et coll. J. Med. Chem. 10, 462 (1967).

Les (pipéridyl-4)-1 benzimidazolinones-2 de formule III sont préparées à partir des (triphénylméthyl-1 pipéridyl-4)-1 benzimidazolinones-2 correspondantes.

La (pipéridyl-4)-1 benzimidazolinone-2 est un produit du commerce.

La présente invention a également pour objet le procédé de préparation des dérivés de formule générale I caractérisé en ce que l'on effectue une réduction alcoylante du mélange de la pipéridylbenzimidazolinone de formule III précédemment définie et d'un phénylglyoxal de formule générale V :

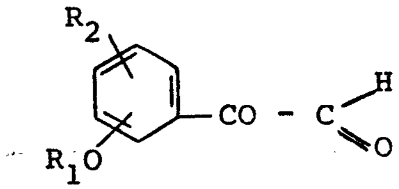 V

dans laquelle :

$R_1$ et $R_2$ ont les significations précédemment définies.

Il est particulièrement avantageux d'effectuer cette réduction alcoylante, selon une méthode analogue à celle décrite par G. FODOR et coll., Am. Soc. 71, 1045 (1949), en opérant sous une pression d'hydrogène comprise entre 5 et 10 atmosphères en présence de catalyseurs choisis parmi les métaux du 8ème groupe, tel que par exemple le platine ou le palladium sur charbon, dans un solvant polaire tel qu'un alcool aliphatique miscible à l'eau, à une température comprise entre 25 et 80°C.

Le ou les groupes protecteurs éventuellement inclus dans le composé V, sont clivés lors de la réduction.

Pour les dérivés acétylés il convient toutefois d'opérer en milieu acide dilué.

Les phénylglyoxals de formule générale V sont soit connus et décrits dans la littérature, soit préparés selon la technique de G. FODOR et coll. Am. Soc. 71, 1045 (1949).

La présente invention a aussi pour objet le procédé de préparation des dérivés de formule générale I caractérisé en ce que l'on fait réagir la pipéridylbenzimidazolinone de formule III précédemment définie avec une halohydrine de formule générale VI :

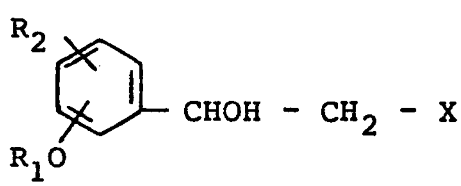 VI

dans laquelle :

X, $R_1$ et $R_2$ sont tels que précédemment définis.

Une telle réaction est effectuée avantageusement dans un solvant polaire tel qu'un alcool aliphatique renfermant 4 ou 5 atomes de carbone, ou le diméthylformamide, à une température comprise entre 110 et 140°C, en présence d'un accepteur de l'hydracide formé au cours de la réaction. Comme accepteurs, on peut employer par exemple les carbonates alcalins tels que le carbonate de sodium ou potassium ou un excès de la pipéridylbenzimidazolinone de formule III.

Le ou les groupes protecteurs éventuellement présents dans le produit de condensation peuvent être ensuite éliminés par les méthodes connues mentionnées précédemment.

Les halohydrines de départ de formule générale VI sont communément préparées par exemple à partir des haloacétophénones de formule générale II correspondantes par réduction au moyen d'un borohydrure alcalin. Elles sont le plus souvent utilisées à l'état brut sans purification.

Les amino alcools de formule générale I obtenus selon les procédés ci-dessus sont des bases faibles amphotères solubles dans les acides et les bases fortes.

La présente invention inclut les sels d'addition des dérivés de formule générale I, notamment ceux obtenus avec des acides forts, et plus particulièrement ceux qui sont physiologiquement tolérables. Parmi les acides utilisables pour la formation de ces sels on peut citer par exemple les acides chlorhydrique, bromhydrique, sulfurique, méthanesulfonique et iséthionique.

Les dérivés de formule générale I et leurs sels physiologiquement tolérables possèdent des propriétés pharmacologiques et thérapeutiques intéressantes notamment des propriétés bronchodilatatrice, β-adrénergique et anti-allergique.

Leur toxicité est faible et leur $DL_{50}$ déterminée chez la souris est supérieure à 1600mg/kg par voie orale ou supérieure à 200mg/kg par voie intrapéritonéale selon les composés.

L'activité bronchodilatatrice a été étudiée chez le cobaye par la méthode de H. Konzett et R. Rossler, Arch. Exp. Path. U. Pharm. 195, 71 (1940). Le dérivé de l'exemple 1, nommé [(dihydroxy-3,4 phényl)-2 hydroxy-2] éthyl-1 (oxo-2 benzimidazolinyl-1)-4 pipéridine, injecté par voie intraveineuse à une dose comprise entre 0,010 et 0,050mg/kg, inhibe totalement le bronchospasme provoqué par l'administration intraveineuse soit d'histamine, soit de sérotonine, soit d'acétylcholine, et l'effet de Slow Reacting Substance, à la dose de 0,050 à 0,100mg/kg. Pour les autres dérivés de l'invention,une telle inhibition totale du bronchospasme est observée pour des doses allant de 0,5 à 10mg/kg IV selon les composés.

Soumis au test de A.K. Armitage, Brit. J. Pharmacol. 17, 196 (1961), les dérivés de l'invention,administrés par voie intrapéritonéale à des doses variant de 0,5 à 20mg/kg selon les composés,inhibent de 50% l'effet produit chez le cobaye par un aérosol d'histamine à 4 %.

Les dérivés de l'invention,administrés par voie intrapéritonéale à des doses de 5 à 20mg/kg,inhibent de 50% le choc anaphylactique provoqué par l'administration d'un aérosol d'albumine à 5% à des cobayes préalablement sensibilisés à l'albumine. Ce test est mis en oeuvre de la façon suivante, sur des lots de 6 cobayes par produit à tester.

Les cobayes sont soumis à une injection intrapéritonéale de 100mg/kg d'ovalbumine en émulsion dans l'adjuvant de Freund. Quatre semaines plus tard, on procède à une sélection des cobayes sensibilisés. Pour celà, les animaux à jeun depuis la veille sont soumis à un aérosol d'ovalbumine à 5% et les temps d'apparition de dyspnée sévère puis de pré-coma sont notés. On sélectionne alors pour les tests suivants, les cobayes, chez lesquels une dyspnée sévère apparaît moins de 3 minutes après le début du traitement à l'aérosol d'ovalbumine. Huit jours plus tard, sur ces cobayes sélectionnés maintenus à jeun depuis la veille, on injecte par voie intrapéritonéale les produits à tester, puis 20 minutes plus tard on soumet alors ces cobayes à un aérosol d'ovalbumine à 5%. On note les temps d'apparition de dyspnée sévère puis de pré-coma, et on détermine ainsi le rôle protecteur des produits testés.

D'autre part, on a observé une action sur l'anaphylaxie cutanée passive provoquée chez le rat selon la technique d'Ovary, Prog. Allergy 5, 459-508, S. Karger Basel / New York, avec les dérivés de l'invention, administrés par voie intraveineuse, à des doses variant de 0,050 à 2,5mg/kg selon les composés.

Les propriétés pharmacologiques ci-dessus décrites, ainsi que la faible toxicité des dérivés de formule générale I et de leurs sels physiologiquement tolérables permettent leur utilisation en thérapeutique notamment dans le traitement de toutes les maladies où il est nécessaire d'inhiber les réactions antigène-anticorps comme les maladies autoimmunes, allergiques et inflammatoires et en particulier celles dans lesquelles un effet β-adrénergique additionnel est bienvenu tel que la dyspnée asthmatique.

La présente invention a également pour objet les compositions pharmaceutiques contenant comme principe actif

un dérivé de formule générale I, ou un de ses sels physiologiquement tolérables, mélangé ou associé à un excipient pharmaceutique approprié, et plus particulièrement à un véhicule convenant à une administration par aérosol.

Les compositions pharmaceutiques ainsi obtenues sont présentées avantageusement sous des formes dosées diverses, telles que par exemple comprimés, dragées, gélules, suppositoires, solutions injectables ou buvables, et plus spécialement sous des formes adaptées à l'administration par aérosol. A titre indicatif, pour le dérivé de l'exemple 1, on peut utiliser des doses de 100 à 750γ de principe actif par bouffée.

Les exemples suivants illustrent l'invention, les points de fusion étant, sauf mention contraire, déterminés au tube capillaire.

EXEMPLE 1 :

[(dihydroxy-3,4 phényl)-2 hydroxy-2] éthyl-1 (oxo-2 benzimidazolinyl-1)-4 pipéridine

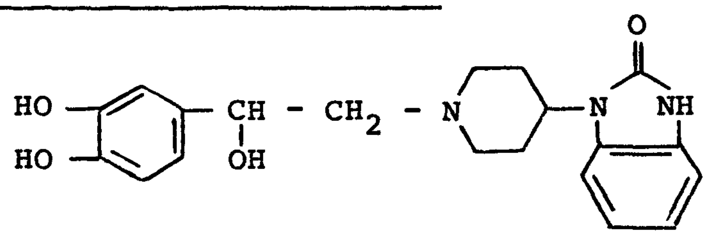

Première méthode :

On chauffe à reflux pendant 12 heures une suspension de 6,87g de dihydroxy-3,4 phényl chlorométhyl cétone, (produit commercial) et de 8g d'(oxo-2 benzimidazolinyl-1)-4 pipéridine, (produit commercial) P.F. 183-185°C, dans 640ml de méthyl éthyl cétone en présence de 1,95g de carbonate de sodium sec et 0,1g d'iodure de sodium. Lorsque la réaction est terminée, le précipité est filtré et lavé à l'eau. Après

séchage, on obtient 14g de base brute que l'on transforme en chlorhydrate dans le méthanol par addition d'éther chlorhydrique. On recueille 8g de cristaux blancs de chlorhydrate de (dihydroxy-3,4 phénacyl)-1 (oxo-2 benzimidazolinyl-1)-4 pipéridine fondant à 310-313°C.

On hydrogène pendant 6 heures une solution de 7g du chlorhydrate ainsi obtenu, dans 770ml d'éthanol à 90%, en présence de 10g de charbon palladié renfermant 10% en poids de palladium, sous une pression d'hydrogène de 70 atmosphères. Lorsque la réduction est terminée, on filtre le catalyseur, évapore le solvant sous pression réduite et traite le chlorhydrate ainsi obtenu par une solution aqueuse bicarbonatée à 10% de Na H $CO_3$. On obtient finalement 2,5g de cristaux beiges de [(dihydroxy-3,4 phényl)-2 hydroxy-2] éthyl-1 (oxo-2 benzimidazolinyl-1)-4 pipéridine, fondant à 216-217°C.

Deuxième méthode :

Une solution de 11,5g de dibenzyloxy-3,4 phényl glyoxal, P.F. 98°C, (préparée selon la technique décrite par G. Fodor et coll. Am. Soc. 71, 1045, 1949), et de 7,25g d'(oxo-2 benzimidazolinyl-1)-4 pipéridine dans 500ml d'éthanol à 90% est soumise à l'hydrogénation sous 5 atmosphères en présence de 5g de charbon palladié renfermant 10% en poids de palladium. Lorsque la quantité théorique d'hydrogène est absorbée, on filtre le caralyseur et évapore le solvant sous pression réduite. Le résidu cristallin est agité avec 2 fois 50ml d'eau bouillante, filtré et dissout dans 100ml d'une solution normale d'acide monométhanesulfonique.

L'insoluble est extrait au chloroforme et la solution aqueuse acide est alcalinisée à pH8 avec du bicarbonate de sodium. La base précipite. Après lavage à l'eau et séchage on obtient 6g de cristaux beiges de [(dihydroxy-3,4 phényl)-2 hydroxy-2] éthyl-1 (oxo-2 benzimidazolinyl-1) -4 pipéridine, fondant à 215-216°C.

Troisième méthode :

On chauffe pendant 8 heures à reflux une solution de 8,4g de (dihydroxy-3,4 phényl)-1 chloro-2 éthanol-1, P.F. 102°C, (préparé à partir de la dihydroxy-3,4 phényl chlorométhyl cétone selon une méthode analogue à celle de O. HINSBERG - brevet allemand n° 364.039 du 16 novembre 1922) et de 10,8g d'(oxo-2 benzimidazolinyl-1)-4 pipéridine dans 250ml de butanol en présence de 6,9g de carbonate de potassium sec.

Lorsque la réaction est terminée on filtre le sel formé et évapore le solvant sous pression réduite. Le résidu est ensuite agité avec 2 fois 50ml d'eau bouillante, filtré et dissout dans 100ml d'acide monométhanesulfonique normal. L'insoluble est extrait au chloroforme et la solution aqueuse acide est alcalinisée à pH8 avec du bicarbonate de sodium. La base précipite. Après lavage à l'eau et séchage on obtient 9g de cristaux beiges de [(dihydroxy-3,4 phényl)-2 hydroxy-2] éthyl-1 (oxo-2 benzimidazolinyl-1)-4 pipéridine, fondant à 215-216°C.

EXEMPLES 2 à 14 :

Les dérivés suivants ont été préparés selon les méthodes décrites dans l'exemple 1 :

2) [(hydroxy-4 phényl)-2 hydroxy-2] éthyl-1 (oxo-2 benzimidazolinyl-1)-4 pipéridine, P.F. 213-215°C (cyanure de méthyle)

3) [(dihydroxy-3,5 phényl)-2 hydroxy-2] éthyl-1 (oxo-2 benzimidazolinyl-1)-4 pipéridine, P.F. de son chlorhydrate : 256-260°C (éthanol)

4) [(hydroxyméthyl-3 hydroxy-4 phényl)-2 hydroxy-2] éthyl-1 (oxo-2 benzimidazolinyl-1)-4 pipéridine, P.F. de son chlorhydrate >360°C (éthanol).

5) [(méthoxycarbonyl-3 hydroxy-4 phényl)-2 hydroxy-2] éthyl-1 (oxo-2 benzimidazolinyl-1)-4 pipéridine, P.F. 206-209°C (méthanol).

6) [(carbamoyl-3 hydroxy-4 phényl)-2 hydroxy-2] éthyl-1 (oxo-2 benzimidazolinyl-1)-4 pipéridine.

7) [(uréido-3 hydroxy-4 phényl)-2 hydroxy-2] éthyl-1 (oxo-2 benzimidazolinyl-1)-4 pipéridine, P.F. 214-218°C (éthanol).

8) [(sulfamoylamino-3 hydroxy-4 phényl)-2 hydroxy-2] éthyl-1 (oxo-2 benzimidazolinyl-1)-4 pipéridine, P.F. de son chlorhydrate : 188°C (éthanol/eau-90/10).

9) [(mésylamino-3 hydroxy-4 phényl)-2 hydroxy-2] éthyl-1 (oxo-2 benzimidazolinyl-1)-4 pipéridine, P.F. de son chlorhydrate : 230-233°C (éthanol).

10) [(éthoxalylamino-3 hydroxy-4 phényl)-2 hydroxy-2] éthyl-1 (oxo-2 benzimidazolinyl-1)-4 pipéridine.

11) [(oxalamino-3 hydroxy-4 phényl)-2 hydroxy-2] éthyl-1 (oxo-2 benzimidazolinyl-1)-4 pipéridine, P.F. 258-260°C (eau).

12) [(formamido-3 hydroxy-4 phényl)-2 hydroxy-2] éthyl-1 (oxo-2 benzimidazolinyl-1)-4 pipéridine, P.F. de son chlorhydrate hemihydrate : 266-270°C (eau).

13) [(dihydroxy-3,4 phényl)-2 hydroxy-2] éthyl-1 (méthyl-3 oxo-2 benzimidazolinyl-1)-4 pipéridine, P.F. 244-246°C, P.F. de son chlorhydrate monohydrate : 178-186°C (éthanol à 95%). La (méthyl-3 oxo-2 benzimidazolinyl-1)-4 pipéridine de départ, P.F. de son carbonate : 145-150°C, a été préparée à partir de la triphénylméthyl-1 (méthyl-3 oxo-2 benzimidazolinyl-1)-4 pipéridine, P.F. 284-289°C, elle-même préparée à partir de la triphénylméthyl-1 (oxo-2 benzimidazolinyl-1) -4 pipéridine, P.F. 292-296°C.

14) [(dihydroxy-3,4 phényl)-2 hydroxy-2] éthyl-1 (allyl-3 oxo-2 benzimidazolinyl-1)-4 pipéridine.

Revendications de brevet

1) Les dérivés de la pipéridylbenzimidazolinone de formule générale I :

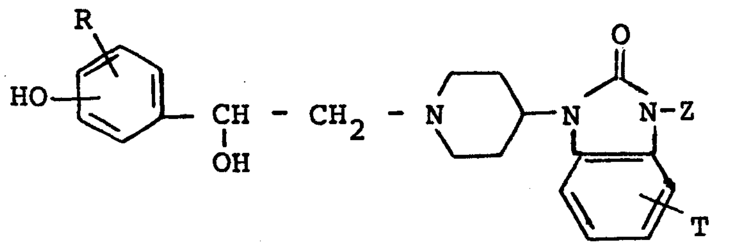

I

dans laquelle :

R représente un atome d'hydrogène, ou un radical hydroxy (OH-), hydroxyméthyle ($HO-CH_2-$), méthoxycarbonyle ($CH_3-O-C(=O)-$), formamido (H-CO-NH-), acétamido ($CH_3-CO-NH-$), mésylamino ($CH_3-SO_2-NH-$), oxalamino (HOOC-CO-NH-), éthoxalylamino ($C_2H_5OOC-CO-NH-$), carbamoyle ($R'R''N-CO-$), uréido ($R'R''N-CO-NH-$) ou sulfamoylamino ($R'R''N-SO_2-NH-$), R' et R" étant dans ces formules identiques ou différents et représentant chacun un atome d'hydrogène ou un radical alkyle ayant de 1 à 5 atomes de carbone,

T représente un atome d'hydrogène ou d'halogène, ou un radical alkyle ou alkoxy ayant chacun de 1 à 5 atomes de carbone, et

Z représente un atome d'hydrogène, ou un radical alkyle ou alcényle chacun renfermant de 1 à 5 atomes de carbone en chaîne droite ou ramifiée.

2) Les sels d'addition des composés de la revendication 1 avec des acides appropriés.

3) Les sels selon la revendication 2 qui sont physiologiquement tolérables.

4) La [(dihydroxy-3,4 phényl)-2 hydroxy-2] éthyl

-1 (oxo-2 benzimidazolinyl-1)-4 pipéridine.

5) L'[(hydroxy-4 phényl)-2 hydroxy-2] éthyl-1 (oxo-2 benzimidazolinyl-1)-4 pipéridine.

6) La [(dihydroxy-3,5 phényl)-2 hydroxy-2] éthyl-1 (oxo-2 benzimidazolinyl-1)-4 pipéridine et son chlorhydrate.

7) Un procédé de préparation des composés de la revendication 1, caractérisé en ce que l'on fait réagir une haloacétophénone de formule générale II

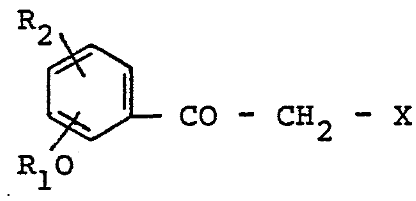

II

dans laquelle :

X représente un atome de chlore ou de brome,

$R_1$ représente un atome d'hydrogène ou un groupe protecteur facilement hydrolysable ou hydrogénolysable et

$R_2$ représente un atome d'hydrogène, ou un radical hydroxy, acétoxy, benzyloxy, hydroxyméthyle, acétoxyméthyle, benzyloxyméthyle, méthoxycarbonyle $(CH_3-O-\underset{\underset{O}{\|}}{C}-)$, formamido (H-CO-NH-), acétamido ($CH_3$-CO-NH-), mésylamino ($CH_3$-$SO_2$-NH-), oxalamino (HOOC-CO-NH-), éthoxalylamino ($C_2H_5$OOC-CO-NH-), carbamoyle ($R'R''$N-CO-), uréido ($R'R''$N-CO-NH-) ou sulfamoylamino ($R'R''$N-$SO_2$-NH-), R' et R" ayant les définitions énoncées dans la revendication 1, sur une pipéridylbenzimidazolinone de formule générale III :

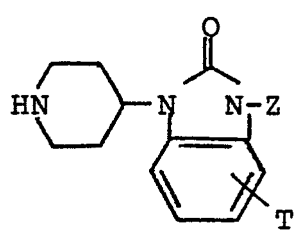

III

dans laquelle :

T et Z ont les significations énoncées dans la revendication 1,

puis réduit le composé ainsi obtenu de formule générale IV :

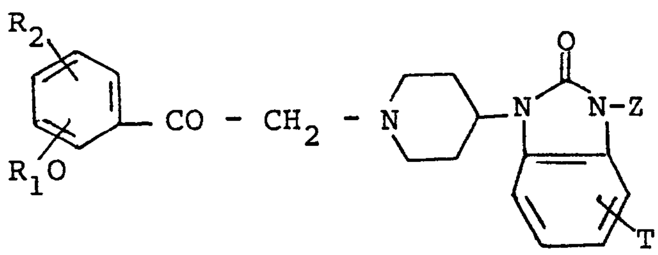

IV

dans laquelle :

$R_1$, $R_2$, T et Z ont les définitions énoncées précédemment et

lorsque le composé réduit renferme des groupes protecteurs, élimine ces derniers selon des méthodes connues.

8) Un procédé de préparation des composés de la revendication 1, caractérisé en ce que l'on effectue une réduction alcoylante du mélange de la pipéridylbenzimidazolinone de formule III définie dans la revendication 7, et d'un phénylglyoxal de formule générale V :

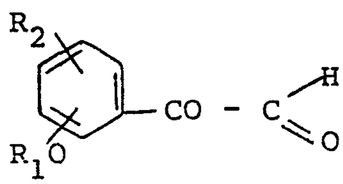

V

dans laquelle :

$R_1$ et $R_2$ ont les significations définies dans la revendication 7.

9) Un procédé de préparation des composés de la revendication 1 caractérisé en ce que l'on fait réagir la pipéridylbenzimidazolinone de formule III définie dans la revendication 7, avec une halohydrine de formule générale VI :

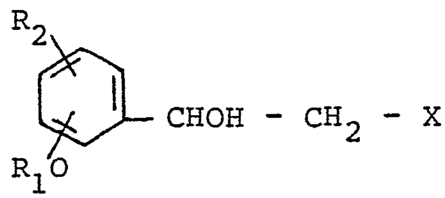

VI

dans laquelle :

X, $R_1$ et $R_2$ sont tels que définis dans la revendication 7, et

lorsque le composé de condensation renferme des groupes protecteurs, élimine ces derniers selon des méthodes connues.

10) Les compositions pharmaceutiques contenant comme principe actif un composé selon les revendications 1 ou 3 à 6 avec les supports pharmaceutiques appropriés.

11) Les compositions pharmaceutiques selon la revendication 10, présentées sous une forme convenant notamment pour le traitement des maladies autoimmunes, allergiques, et inflammatoires et pour le traitement de l'insuffisance respiratoire de l'asthme.

Office européen des brevets

# RAPPORT DE RECHERCHE EUROPEENNE

0008259

Numéro de la demande

EP 79 40 0509

| Catégorie | DOCUMENTS CONSIDERES COMME PERTINENTS<br>Citation du document avec indication, en cas de besoin, des parties pertinentes | Revendication concernée | CLASSEMENT DE LA DEMANDE (Int. Cl. 3) |
|---|---|---|---|
| | FR - A - 1 469 467 (JANSSEN)<br>* Revendication; page 1; colonne de droite *<br>-- | 1,7,9, 10,11 | C 07 D 401/04<br>A 61 K 31/445 |
| | FR - A - 2 213 059 (JANSSEN)<br>* Revendication *<br>---- | 1,7,9, 10,11 | |

DOMAINES TECHNIQUES RECHERCHES (Int. Cl. 3)

C 07 D 401/04
A 61 K 31/445

CATEGORIE DES DOCUMENTS CITES

X: particulièrement pertinent
A: arrière-plan technologique
O: divulgation non-écrite
P: document intercalaire
T: théorie ou principe à la base de l'invention
E: demande faisant interférence
D: document cité dans la demande
L: document cité pour d'autres raisons

&: membre de la même famille, document correspondant

X Le présent rapport de recherche a été établi pour toutes les revendications

| Lieu de la recherche | Date d'achèvement de la recherche | Examinateur |
|---|---|---|
| La Haye | 14-11-1979 | CREMERS |

OEB Form 1503.1 06.78